Europäisches Patentamt

European Patent Office

Office européen des brevets

(11) Veröffentlichungsnummer: **0 297 444**
**A2**

(19)

(12) **EUROPÄISCHE PATENTANMELDUNG**

(21) Anmeldenummer: 88110064.8

(22) Anmeldetag: 24.06.88

(51) Int. Cl.⁴: **C07C 29/10 , C07C 31/22**

(30) Priorität: 30.06.87 DE 3721495

(43) Veröffentlichungstag der Anmeldung:
04.01.89 Patentblatt 89/01

(84) Benannte Vertragsstaaten:
**BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Eggersdorfer, Manfred, Dr.**
**Hannongstrasse 18**
**D-6710 Frankenthal(DE)**
Erfinder: **Siegel, Hardo, Dr.**
**Hans-Purrmann-Allee 25**
**D-6720 Speyer(DE)**
Erfinder: **Mueller, Herbert, Dr.**
**Carostrasse 53**
**D-6710 Frankenthal(DE)**
Erfinder: **Pohl, Hans Henning, Dr.**
**Mandelring 221**
**D-6730 Neustadt(DE)**

(54) **Verfahren zur Herstellung von 1,2,4-Butantriol.**

(57) Verfahren zur Herstellung von 1,2,4-Butantriol, bei dem man 1,2,4-Butantriol in der wünschenswerten technisch einfachen und wirtschaftlichen Weise herstellen kann, indem man But-3-en-1-ol in einem Lösungsmittel bei Temperaturen bis 100 °C in Gegenwart von Säuren oder Oxiden der Metalle der Gruppen IV, V, VI oder VIII mit Wasserstoffperoxid umsetzt und das dabei gebildete 1,2-Epoxi-butan-4-ol bei höherer Temperatur hydrolysiert.

EP 0 297 444 A2

## Verfahren zur Herstellung von 1,2,4-Butantriol

Die Erfindung betrifft ein neues Verfahren zur Herstellung von 1,2,4-Butantriol durch Epoxidierung von But-3-en-1-ol zu 1,2-Epoxy-butan-4-ol und nachfolgende Hydrolyse.

1,2,4-Butantriol ist ein wichtiges Zwischenprodukt, das z.B. für die Herstellung von Pharmazeutika, von Polyurethanen sowie als Glycerinersatz Verwendung findet. Für alle diese Anwendungen wird 1,2,4-Butantriol von hoher Reinheit gefordert.

In der japan. Offenlegungsschrift 70632/84 wird ein Verfahren zur Herstellung von 1,2,4-Butantriol beschrieben, bei dem man Buten-1,4-diol oxidiert und das gebildete 2,3-Epoxi-butan-1,4-diol in einem wasserhaltigen organischen Lösungsmittel an einem Pd-Katalysator hydriert. Nachteilig an diesem Verfahren ist, daß bei der Oxidation in wäßrigem Milieu bereits eine Hydrolyse des Epoxids eintritt, wodurch die Ausbeute gemindert und zugleich die Reinheit des Produkts durch Bildung von 1,2,3,4-Butantetrol erheblich beeinträchtigt wird.

Aus der US-PS 2 657 242 ist bekannt, daß man 1,2,4-Butantriol durch Hydratisierung von 1,4-Butindiol und nachfolgende Hydrierung des so gewonnenen Butan-1,4-diol-2-ons herstellen kann. Dieses Verfahren hat den Nachteil, daß man ein Produkt erhält, das durch Butan-1,4-diol-2-on verunreinigt ist.

Es hat deshalb nicht an Versuchen gefehlt, die Bildung von unerwünschten Nebenprodukten bei der Herstellung von 1,2,4-Butantriol auszuschließen. So wird in DP-PS 1 024 496 vorgeschlagen, an But-3-en-1-ol-formiat in Isopropylformiat Wasserstoffperoxid anzulagern, anschließend Isopropylformiat, Wasser, Wasserstoffperoxid und Ameisensäure abzudestillieren und das Anlagerungsprodukt durch Umesterung in 1,2,4-Butantriol zu überführen. Die Ausbeute dieses Verfahrens liegt bei 70 bis 75 %. Das Verfahren vermeidet zwar die Möglichkeit der Bildung von 1,2,3,4-Butantetrol, seine Ausübung im technischen Maßstab ist jedoch sehr aufwendig und erfordert mehrere Verfahrensschritte.

Der Weg der direkten Epoxidierung von But-3-en-1-ol erscheint aufgrund des Standes der Technik als nicht aussichtsreich. Zwar wird in J. Chem. Soc. (1957), 4608-4612 die Epoxidierung von But-3-en-1-ol mit Perbenzoesäure beschrieben, doch ist die Ausbeute unbefriedigend und das Verfahren wegen der Notwendigkeit, Perbenzoesäure in molaren Mengen als Oxidationsmittel einzusetzen, auf den Labormaßstab beschränkt. In Houben-Weyl "Methoden der organischen Chemie" Bd. VI/3 (1965) S. 371-487 werden verschiedene Methoden der Epoxidierung von Olefinen aufgezeigt und deren Anwendungsbreite und -grenzen diskutiert. So wird die Reaktionsgeschwindigkeit der Epoxidierung (siehe S. 392-393) durch Art und Zahl der Substituenten, die sich an der Ethylenbindung befinden, stark beeinflußt. Elektronenanziehende Gruppen erschweren die Reaktion, Alkylsubstituenten erhöhen die Reaktionsgeschwindigkeit. Dies bedeutet auch, daß Olefine mit endständiger Doppelbindung wie dies bei But-3-en-1-ol der Fall ist, viel schwerer als solche mit mittelständiger Doppelbindung reagieren.

Es wurde nun überraschenderweise gefunden, daß man 1,2,4-Butantriol in der wünschenswerten technisch einfachen und wirtschaftlichen Weise herstellen kann, wenn man But-3-en-1-ol in einem Lösungsmittel bei Temperaturen von 20 bis 100°C in Gegenwart von Säuren oder Oxiden der Metalle der Gruppen IV, V, VI oder VIII mit Wasserstoffperoxid umsetzt und das dabei gebildete 1,2-Epoxibutan-4-ol bei höherer Temperatur hydrolysiert.

But-3-en-1-ol ist z.B. durch Umsetzung von Propen und Formaldehyd (JACS 77 (1955) 4666) oder durch Esterpyrolyse von 1,3-Dihydroxybutanformiat (DP 1 024 496) und nachfolgende Hydrolyse herstellbar.

Man setzt das But-3-en-1-ol mit dem Wasserstoffperoxid in einem Lösungsmittel um, wobei man 1,0 bis 2,0, vorzugsweise 1,1 bis 1,4 Mol Wasserstoffperoxid per Mol But-3-en-1-ol anwendet. Das Wasserstoffperoxid wird bevorzugt als wäßrige Lösung eingesetzt, wobei die Wasserstoffperoxid-Konzentration zweckmäßigerweise über 30, vorzugsweise zwischen 50 und 80 Gew.% liegt. Man kann zwar auch niedriger konzentrierte Lösungen verwenden, jedoch liegen dann die Reaktionszeiten hoch. Zudem erfordert ein Arbeiten mit niedriger konzentrierten Lösungen einen Überschuß an eingesetztem Wasserstoffperoxid, bezogen auf das Olefin, weil sich aufgrund der längeren Reaktionszeit ein erheblicher Teil des Wasserstoffperoxids ohne die gewünschte Reaktion einzugehen, zersetzt. Höher konzentriertes Wasserstoffperoxid kann ebenfalls Verwendung finden, allerdings müssen dann Sicherheitsmaßnahmen wegen der hohen Zersetzungsgefahr ergriffen werden.

Man verfährt beispielsweise so, daß man die Lösung von But-3-en-1-ol in einem Lösungsmittel vorlegt und zu dieser Lösung portionsweise oder kontinuierlich Wasserstoffperoxid oder die Wasserstoffperoxidlösung gibt. Als Lösungsmittel für das Olefin kommen solche in Betracht, die unter den Umsetzungsbedingungen mit den Reaktionspartnern nicht reagieren; das sind z.B. Chloroform, Chlortoluol, Toluol, Dimethylformamid und Wasser, von denen Wasser bevorzugt ist. Die Konzentration des Olefins in der Ausgangslösung kann in weiten Grenzen eingestellt werden, zweckmäßigerweise verwendet man eine 30 bis 70 gewichtsprozente Lösung des Olefins. Beispielsweise gibt man das Wasserstoffperoxid oder die Wasserstoffperoxidlösung im Verlauf von 0,1 bis zu mehreren Stunden portionsweise oder kontinuierlich in die vorgelegte Lösung des Olefins. Die Zugabezeit ist abhängig von der Reaktionstemperatur. Üblicherweise wird in einem Zeitraum von 0,5 bis 2 h zugegeben.

Die Epoxidierung wird bei Temperaturen von 10 bis 100, vorzugsweise bei 30 bis 100° C durchgeführt. Das Olefin wird mit dem Wasserstoffperoxid in Gegenwart von Säuren oder Oxiden der Metalle der Gruppen IV, V, VI oder VIII des Periodensystems umgesetzt. Die Konzentration dieser Katalysatoren beträgt z.B. 0,01 bis 5 Gew.%, bezogen auf das Reaktionsgemisch. Höhere Konzentrationen sind zwar möglich, bieten allerdings keine besonderen Vorteile.

Geeignete Säuren sind z.B. Wolframsäure, Chromsäure oder Heteropolysäuren, wie Wolfram-selen-säuren, Wolfram-Schwefelsäure oder Molybdänschwefelsäure. Als Oxide von Metallen der Gruppen IV, V, VI oder VIII kommen z.B. die Oxide von Titan, Vanadin, Chrom, Molybdän, Wolfram und Ruthenium in Betracht.

Nach einer besonders vorteilhaften Arbeitsweise führt man die Umsetzung in einem pH-Bereich von 4 bis 7, insbesondere bei einem pH von 5 bis 6,8, durch. Zu diesem Zweck gibt man, falls erforderlich, basisch wirkende Stoffe, wie Natriumhydroxid, Kaliumhydroxid, Natriumcarbonat oder Calciumhydroxid, zweckmäßigerweise als wäßrige Lösung zum Reaktionsgemisch. Die Reaktionszeit hängt stark von den gewählten Reaktionsbedingungen ab. So fallen die Reaktionszeiten kürzer aus, wenn man bei höherer Temperatur und hohen Konzentrationen an Katalysator und Wasserstoffperoxid arbeitet und umgekehrt.

Das nach dem erfindungsgemäßen Verfahren zunächst entstandene 1,2-Epoxy-butan-4-ol kann man vor der Hydrolyse isolieren oder durch weitere Umsetzung des Reaktionsgemisches hydrolysieren. Will man das 1,2-Epoxy-butan-4-ol isolieren und reinigen, so verfährt man z.B. so, daß man das 1,2-Epoxy-butan-4-ol aus dem Reaktionsgemisch mit Lösungsmitteln, wie Methylenchlorid, Chloroform oder Toluol extrahiert oder daß man das Reaktionsgemisch neutralisiertund die organische Phase fraktioniert destilliert. Man hydrolysiert das 1,2-Epoxy-butan-4-ol nach an sich bekannten Methoden bei Temperaturen von 50 bis 160° C, z.B. indem man es unter milden basischen oder sauren Bedingungen auf Temperaturen von 50 bis 130° C erhitzt. Man kann das Epoxid aber auch in neutralem Medium durch Erhitzen auf Temperaturen von 90 bis 160° C in das 1,2,4-Butantriol überführen.

Die Arbeitsweise, bei der man das Reaktionsgemisch der ersten Stufe ohne Isolierung des 1,2-Epoxy-butan-4-ol· der Hydrolyse unterwirft, ist bevorzugt. Bei saurer Hydrolyse gibt man Säuren, vorzugsweise in Wasser lösliche Säuren, wie Salzsäure, Schwefelsäure, Phosphorsäure, Bromwasserstoff, Überchlorsäure, schweflige Säure, Ameisensäure oder Essigsäure zu. Will man basisch hydrolysieren, so gibt man basisch wirkende Stoffe, wie Natriumhydroxid, Kaliumhydroxid, Natriumcarbonat, Calciumhydroxid oder Amine zu. Im allgemeinen werden nur katalytische Mengen Säure bzw. Base verwendet. Die Menge an Säure bzw. Base wird so gewählt, daß man einen pH-Wert zwischen 3 und 11 einstellt. Die Hydrolyse wird bei erhöhter Temperatur, vorzugsweise im Bereich von 50 bis 100° C ausgeführt. Nach erfolgter Hydrolyse wird die erhaltene Lösung neutralisiert und destillativ aufgearbeitet.

Das neue Verfahren zeichnet sich durch einfache Durchführbarkeit aus und liefert 1,2,4-Butantriol in hoher Reinheit und Ausbeute.

Die in den Beispielen genannten Prozente sind Gewichtsprozente.

Beispiel 1

In einem Rührkolben werden 3,5 g H₂WO₄, 150 ml Wasser und 72 g But-3-en-1-ol vorgelegt. Unter Rühren werden langsam 100 g 50 %ige wäßrige H₂O₂-Lösung zugegeben. Die Zugabe ist nach 15 bis 30 Minuten beendet. Während dieses Vorgangs wird der pH-Wert kontrolliert und durch Zugabe von verdünnter wäßriger NaOH-Lösung im pH-Bereich von 4,0 bis 7,0 gehalten.

Die Reaktionslösung erwärmt sich auf etwa 50° C. Sie wird nach erfolgter Zugabe noch 2 Stunden durch Heizen auf dieser Temperatur gehalten. Dann rührt man das Reaktionsgemisch noch weitere 8 h bei 30° C.

Das Reaktionsgemisch wird mit Natronlauge neutralisiert und destillativ aufgearbeitet. Zunächst destilliert man bei 10 bis 20 mbar das Wasser ab. Der organische Anteil wird anschließend bei 3 bis 7 mbar fraktioniert. Man erhält 77,5 g 1,2-Epoxy-butan-4-ol, das entspricht einer Ausbeute von 88 %, bezogen auf Butenol.

Das erhaltene 1,2-Epoxy-butan-4-ol wird in 100 g Wasser gelöst. Man gibt 0,5 ml 5n-Schwefelsäure zu und rührt 3 Stunden bei 95° C. Anschließend wird mit Natronlauge neutralisiert, dann wird bei atmosphärischem Druck das Wasser abdestilliert. Der organische Anteil wird bei 2 mbar destilliert. Man erhält 82 g (93 % d. Th.) 1,2,4-Butantriol.

Beispiel 2

Man verfährt wie in Beispiel 1 beschrieben, wobei man das 1,2-Epoxybutan-4-ol jedoch nicht isoliert, sondern das Reaktionsgemisch bei pH 6 für 3 Stunden bei 80°C rührt. Das Reaktionsgemisch wird wie in Beispiel 1 beschrieben aufgearbeitet. Man erhält 94,5 g 1,2,4-Butantriol (= 89 % d. Th., bezogen auf Butenol).

Beispiel 3

Man arbeitet wie in Beispiel 2 beschrieben, wobei man das das 1,2-Epoxy-butan-4-ol enthaltende Reaktionsgemisch nach Zugabe von 0,5 ml 5n-Natronlauge 3 Stunden auf 90°C erhitzt. Man erhält 95,8 g 1,2,4-Butantriol (= 90,4 % d. Th., bezogen auf Butenol).

**Ansprüche**

1. Verfahren zur Herstellung von 1,2,4-Butantriol, dadurch gekennzeichnet, daß man But-3-en-1-ol in einem Lösungsmittel bei Temperaturen bis 100°C in Gegenwart von Säuren oder Oxiden der Metalle der Gruppen IV, V, VI oder VIII mit Wasserstoffperoxid umsetzt und das dabei gebildete 1,2-Epoxi-butan-4-ol bei höherer Temperatur hydrolysiert.

2. Verfahren nach Anspruch 1, dadurch gekennzeichnet, daß man das But-3-en-1-ol in wäßriger Lösung mit Wasserstoffperoxid umsetzt.

3. Verfahren nach Anspruch 1, dadurch gekennzeichnet, daß man die Umsetzung zum Epoxid bei Temperaturen von 10 bis 100°C vornimmt.

4. Verfahren nach Anspruch 1, dadurch gekennzeichnet, daß man Säuren oder Oxide von Titan, Vanadin, Chrom, Molybdän oder Ruthenium verwendet.

5. Verfahren nach Anspruch 1, dadurch gekennzeichnet, daß man die Umsetzung des But-3-en-1-ol mit Wasserstoffperoxid im pH-Bereich von 4 bis 7 durchführt.

6. Verfahren nach Anspruch 1, dadurch gekennzeichnet, daß man die Hydrolyse des 1,2-Epoxi-butan-4-ol bei einem pH-Wert von 3 bis 11 und bei Temperaturen von 50 bis 100°C durchführt.